# EUROPEAN PATENT APPLICATION

(11) **EP 0 685 230 A1**
(43) Date of publication of application: **06.12.1995**
(21) Application number: 95902982.8
(22) Date of filing: 19.12.1994
(51) Int. Cl.: A61K 31/34, A61K 31/365, A61K 9/06, A61K 47/18

(54) **STABLE DERMATOLOGIC PREPARATION CONTAINING MYCOPHENOLIC ACID**

(30) Priority: 22.12.1993 JP 323851/93
(71) Applicant: TOKO YAKUHIN KOGYO KABUSHIKI KAISHA, Osaka-shi, Osaka 530 (JP)
(72) Inventor: KAMISHITA, Takuzo, Osaka 569 (JP); MIYAZAKI, Takashi, Toyama 930-03 (JP)
(74) Representative: Weisert, Annekäte, Dipl.-Ing. Dr.-Ing.
(86) International application number: JP9402131
(87) International publication number: WO9517179

(57) **Abstract**

A stable dermatologic preparation which is efficacious in treating various skin deseases such as psoriasis and contains mycophenolic acid as the active ingredient together with at least one stabilizers selected from among crotamiton, methyl salicylate and glycol salicylate.

## Description

### Technical Field

This invention relates to a pharmaceutical composition for the epidermic application which contains as an active ingredient mycophenolic acid stably. More particularly, it relates to a stable pharmaceutical composition for the epidermic application which comprises as an active ingredient mycophenolic acid and one or more of an oily liquid substance selected from crotamiton, methyl salicylate and glycol salicylate.

### Background Art

Mycophenolic acid has been found at the end of 19 century, i.e. in 1896, and it is known that the compound is one of the substances produced by a microbe and has an isobenzofuran nucleus. The pharmacological activities thereof has recently been found and it is known to have biological activities such as "anti-tumor activity", "immunosuppressive activity" (Japanese Patent Second Publication (Kokoku) No. 47-7955). It is disclosed in a publication gazette of said Japanese patent that it was experimentally confirmed that it was useful for the treatment of malignant tumor and psoriasis in the form of a preparation for oral administration or an injection preparation.

On the other hand, it has been reported that immunosuppressive drugs specific to T-cells are useful for the treatment of dermatic diseases such as psoriasis in the form of a preparation for oral administration or an injection preparation, and some drugs have practically been used for such a purpose. It has also been studied to use them in the form of a composition for epidermic application in order to exhibit their effects topically and effectively. From this viewpoint, it may also be considered that mycophenolic acid having similar immunosuppressive activity may be applied topically, i.e. percutaneously in order to treat various skin diseases such as psoriasis, atopic dermatitis, alopecia areata, and it has been desired to find a pharmaceutical composition which is effective to transport the active mycophenolic acid to the diseased part in a high concentration and to exhibit effectively its activities at the diseased part.

It is reported that even if an immunosuppressive drug shows high activities by oral administration or by injection, it can not exhibit any activity against, for example, psoriasis when applied by percutaneous route (cf. Lancet, 1987, p. 806; and Journal of Investigative Dermatology, Vol. 90, p. 251, 1988). Thus, there are various problems for preparing an epidermic composition of mycophenolic acid. For example, mycophenolic acid is rather easily soluble in methanol or ethanol but is disadvantageously almost insoluble in water and other organic solvents (cf. Japanese Patent Second Publication (Kokoku) No. 48-1074), and hence, it shall be dissolved in water after being converted into an alkali salt by treating it with sodium hydroxide, etc. (cf. Japanese Patent Second Publication (Kokoku) No. 47-7955, Example 2). However, when an epidermic composition is prepared by this method, the composition has some problems, such as unstability of the active mycophenolic acid with time, and further crystallization thereof and brocking and hence, the composition is not suitable from the practical viewpoints.

Thus, when mycophenolic acid is dissolved in water by using sodium hydroxide etc. for the purpose of preparing a pharmaceutical composition for the epidermic application useful for the treatment of psoriasis, atopic dermatitis, alopecia areata, etc., it has disadvantageously less stability with time, and hence, it is required to find an improved composition having good stability with time and good penetration into the skin.

### Description of the Invention

In order to eliminating the defects in the known compositions containing mycophenolic acid, the present inventors have studied to find the most suitable solvent for dissolving mycophenolic acid and further a composition having good stability with time and good penetration into the skin, and have found that when a composition of mycophenolic acid is prepared by using one or more of an oily liquid substance selected from crotamiton, methyl salicylate and glycol salicylate, there can be obtained the desired composition having a good stability with time, and then, the present invention has been completed.

The present invention provides a pharmaceutical composition for the epidermic application which can keep the active mycophenolic acid stably by incorporating one or more of a stabilizer selected from crotamiton, methyl salicylate, and glycol salicylate.

The pharmaceutical composition for the epidermic application of the present invention contains as the active ingredient mycophenolic acid in an amount of 0.01 to 5.0 % by weight, preferably 0.1 to 2.0 % by weight, based on the whole weight of the composition, which is prepared by dissolving the active compound with one or more of a stabilizer selected from crotamiton, methyl salicylate and glycol salicylate, and incorporating the solution into a conventional base for epidermic composition suitable for the desired form of composition. It is usually prepared by previously dissolving mycophenolic acid in crotamiton, methyl salicylate and/or glycol salicylate and then incorporating the solution into a base suitable for pharmaceutical composition for the epidermic application. However, instead thereof, mycophenolic acid may be directly added to a base material for the epidermic composition, and simultaneously or thereafter, crotamiton, methyl salicylate and/or glycol salicylate are(is) added thereto, and finally, the mixture is formed into the desired form of a preparation by a conventional method.

The crotamiton, methyl salicylate and glycol salicylate to be used for stabilizing mycophenolic acid can very easily dissolve mycophenolic acid and can keep it very stably. When other oily liquid substances, such as higher fatty acid esters (e.g. isopropyl myristate, etc.), higher alcohols (e.g. 2-octyldodecanol, etc.), or liquid paraffins are used alone, it is difficult to obtain the desired stable composition. These crotamiton, methyl salicylate and glycol salicylate are used in an amount sufficient for dissolving mycophenolic acid, and the most suitable amount thereof may vary depending on the kinds of preparations, but is usually in the range of 1 to 100 parts by weight, preferably 1 to 50 parts by weight, more preferably 1 to 25 parts by weight, per 1 part by weight of mycophenolic acid, and the amount is in the range of 1.0 to 15.0 % by weight based on the whole weight of the composition.

The mycophenolic acid-containing pharmaceutical composition for the epidermic application of the present invention may be formulated in any conventional preparation for epidermic application, such as ointment, cream, gel, gel cream, lotion, solution, and the like. These preparations may be prepared by a conventional method by dissolving the active mycophenolic acid in crotamiton, methyl salicylate and/or glycol salicylate and incorporating it into a conventional base for epidermic preparations.

For example, in the preparation of an ointment, vaseline, waxes, paraffins, vegetable oils, plastibase, polyethylene glycol, etc. are used.

In the preparation of a cream preparation, fats and oils, waxes, higher fatty acids, higher alcohols, fatty acid esters, purified water, polyvalent alcohols, emulsifying agents etc. are used.

In the preparation of a gel preparation, there are used a carboxyvinyl polymer, a water-soluble basic compound (e.g. alkali metal hydroxides, alkanolamines, etc.), hydroxypropyl cellulose, hydroxypropyl methyl cellulose, polyvinyl alcohol, polyvinylpyrrolidone, purified water, lower alcohols, polyvalent alcohols, polyethylene glycol, and the like.

In the preparation of a gel cream preparation, an emulsifying agent (preferably nonionic surfactants), an oily liquid substance (e.g. liquid paraffin, isopropyl myristate, 2-octyldodecanol, etc.), and the like are used in addition to the above gelling substrate.

In the preparation of lotion and solution preparations, lower alcohols (e.g. ethanol, isopropanol, etc.) and polyvalent alcohols (e.g. glycerin, propylene glycol, 1,3-butylene glycol, etc.), and purified water are used.

In any of the above preparations, squalane, cholesterols, lecithin, and the like may optionally be added in addition to the above-mentioned ingredients. Moreover, any other conventional additives to be usually incorporated into the epidermic preparations, such as antioxidants, preservatives, humectants, chelating agents, and the like may also be incorporated. Those preparations may be prepared under the conventional conditions for the conventional epidermic preparations.

The composition of the present invention is illustrated by the following Examples and Experiments.

### Example 1 (Ointment)

An ointment is prepared by the following formulation.

| (Components) | (Amount) |
|---|---|
| Mycophenolic acid | 0.1 |
| Crotamiton | 10.0 |
| White vaseline | 89.9 |
| | Totally 100.0 g |

White vaseline is dissolved by warming on a water bath, and thereto is added a solution of mycophenolic acid in crotamiton which is prepared by warming at about 70 to 80°C, and the mixture is well stirred, and thereafter, cooled and kneaded until it becomes semisolid to give an ointment.

### Example 2 (Ointment)

An ointment is prepared by the following formulation.

| (Components) | (Amount) |
|---|---|
| Mycophenolic acid | 0.2 |
| Glycol salicylate | 5.0 |
| Castor oil | 5.0 |
| Glycerin monostearate | 2.0 |
| White vaseline | 87.8 |
| | Totally 100.0 g |

White vaseline is dissolved by warming on a water bath, and thereto is added a solution of mycophenolic acid in crotamiton which is prepared by warming at about 70 to 80°C, and the mixture is well stirred, and thereafter, cooled and kneaded until it becomes semisolid to give an ointment.

### Example 3 (Ointment)

An ointment is prepared by the following formulation.

| (Components) | (Amount) |
|---|---|
| Mycophenolic acid | 2.0 |
| Glycol salicylate | 15.0 |
| Polyethylene glycol 4000 | 50.0 |
| Polyethylene glycol 400 | 33.0 |
| | Totally 100.0 g |

Polyethylene glycol 4000 and polyethylene glycol 400 are dissolved by warming at 60°C on a water bath, and thereto is added a solution of mycophenolic acid in glycol salicylate which is prepared by warming at about 70 to 80°C, and the mixture is well stirred, and thereafter, cooled and kneaded until it becomes semisolid to give an ointment.

### Example 4 (Ointment)

An ointment is prepared by the following formulation.

| (Components) | (Amount) |
|---|---|
| Mycophenolic acid | 1.0 |
| Crotamiton | 5.0 |
| Plastibase | 94.0 |
| | Totally 100.0 g |

To plastibase is added a solution of mycophenolic acid in crotamiton which is prepared by warming at about 70 to 80°C, and the mixture is well stirred and kneaded to give an ointment.

### Example 5 (Cream preparation)

A cream preparation is prepared by the following formulation.

| (Components) | (Amount) |
|---|---|
| Mycophenolic acid | 0.5 |
| Glycol salicylate | 5.0 |
| White vaseline | 25.0 |
| Stearyl alcohol | 25.0 |
| 1,3-Butylene glycol | 10.0 |
| Sodium laurylsulfate | 1.5 |
| Polyoxyl 40 stearate | 2.0 |
| Methyl parahydroxybenzoate | 0.025 |
| Propyl parahydroxybenzoate | 0.015 |
| Purified water | 30.96 |
| | Totally 100.0 g |

Mycophenolic acid is dissolved in glycol salicylate at about 70 to 80°C. Thereto are added white vaseline and stearyl alcohol, and the mixture is dissolved and well stirred on a water bath and kept at about 70 to 80°C on the water bath. To the mixture is added a solution of other components which is previously prepared by dissolving them in purified water and warming at about 70 to 80°C. The mixture is well mixed and stirred until it becomes semisolid to give a cream preparation.

### Example 6 (Cream preparation)

A cream preparation is prepared by the following formulation.

| (Components) | (Amount) |
|---|---|
| Mycophenolic acid | 0.5 |
| Crotamiton | 2.0 |
| Glycol salicylate | 3.0 |
| White vaseline | 15.0 |
| Stearyl alcohol | 13.0 |
| Purified lanolin | 15.0 |
| Propylene glycol | 12.0 |
| Sorbitan sesqui-oleate | 2.0 |
| Polyoxyl 40 stearate | 5.0 |
| Methyl parahydroxybenzoate | 0.025 |
| Propyl parahydroxybenzoate | 0.015 |
| Purified water | 32.46 |
| | Totally 100.0 g |

Mycophenolic acid is dissolved in crotamiton and glycol salicylate at about 70 to 80°C. Thereto are added white vaseline, stearyl alcohol, and purified lanolin, and the mixture is dissolved and well stirred on a water bath and kept at about 70 to 80°C on the water bath. To the mixture is added a solution of other components which is previously prepared by dissolving them in purified water and warming at about 70 to 80°C. The mixture is well mixed and stirred until it becomes semisolid to give a cream preparation.

### Example 7 (Cream preparation)

A cream preparation is prepared by the following formulation.

| (Components) | (Amount) |
|---|---|
| Mycophenolic acid | 0.25 |
| Crotamiton | 5.0 |
| White vaseline | 40.0 |
| Cetanol | 18.0 |
| Sorbitan sesqui-oleate | 5.0 |
| Lauromacrogol | 0.5 |
| Methyl parahydroxybenzoate | 0.1 |
| Propyl parahydroxybenzoate | 0.1 |
| Purified water | 31.05 |
| | Totally 100.0 g |

Mycophenolic acid is dissolved in crotamiton at about 70 to 80°C, and thereto are added white vaseline, cetanol, sorbitan sesqui-oleate, lauromacrogol, and propyl parahydroxybenzoate, and the mixture is dissolved and well stirred on a water bath and kept at about 70 to 80°C on the water bath. To the mixture is added gradually a solution of methyl parahydroxybenzoate in purified water which is previously prepared by warming at about 80°C. The mixture is well mixed and stirred until it becomes semisolid to give a cream preparation.

### Example 8 (Gel preparation)

A gel preparation is prepared by the following formulation.

| (Components) | (Amount) |
|---|---|
| Mycophenolic acid | 1.0 |
| Crotamiton | 5.0 |
| Polyethylene glycol 400 | 20.0 |
| 1,3-Butylene glycol | 40.0 |
| 4 % Aqueous carboxyvinyl polymer solution | 26.0 |
| 4 % Aqueous sodium hydroxide solution | 8.0 |
| | Totally 100.0 g |

Mycophenolic acid is dissolved in crotamiton, polyethylene glycol 400 and 1,3-butylene glycol by stirring well. The above mycophenolic acid-containing solution is added to a gel substrate which is previously prepared by gelling a 4 % aqueous carboxyvinyl polymer solution with a 4 % aqueous sodium hydroxide, and the mixture is homogeneously stirred to give a gel preparation.

### Example 9 (Gel preparation)

A gel preparation is prepared by the following formulation.

| (Components) | (Amount) |
|---|---|
| Mycophenolic acid | 0.5 |
| Glycol salicylate | 5.0 |
| Ethanol | 53.4 |
| 1,3-Butylene glycol | 15.0 |
| 4 % Aqueous carboxyvinyl polymer solution | 25.0 |
| Hydroxypropylmethyl cellulose | 1.0 |
| Triethanolamine | 0.1 |
| | Totally 100.0 g |

Mycophenolic acid is dissolved in a mixture of glycol salicylate, a part of ethanol and 1,3-butylene glycol by stirring well. Separately, to a homogeneous mixture of hydroxypropylmethyl cellulose in a part of ethanol is added a 4 % aqueous carboxyvinyl polymer solution and stirred, and thereto is added triethanolamine, and the mixture is made homogeneous by stirring to give a gel base. To the gel base is added the above mycophenolic acid-containing solution, and the mixture is homogeneously stirred to give a gel preparation.

### Example 10 (Gel cream preparation)

A gel cream preparation is prepared by the following formulation.

| (Components) | (Amount) |
|---|---|
| Mycophenolic acid | 0.5 |
| Crotamiton | 5.0 |
| 2-Octyldodecanol | 10.0 |
| 1,3-Butylene glycol | 5.0 |
| Glycerin monostearate | 0.5 |
| Polyoxyl 40 monostearate | 0.5 |
| 4 % Aqueous carboxyvinyl polymer solution | 25.0 |
| 1 % Aqueous sodium hydroxide solution | 9.0 |
| Purified water | 44.5 |
| | Totally 100.0 g |

Mycophenolic acid is dissolved in crotamiton, 2-octyldodecanol, glycerin monostearate and polyoxyl 40 monostearate at about 70 to 80°C. Separately, to a 4 % aqueous carboxyvinyl polymer solution are added a 1 % aqueous sodium hydroxide solution, 1,3-butylene glycol and purified water, and the mixture is stirred to give a homogeneous gel and thereafter, the mixture is warmed at about 70 to 80°C. To the mixture is added the above-prepared warmed solution of the active ingredient-containing solution, and the mixture is emulsified and homogeneously mixed to give a gel cream preparation.

### Example 11 (Gel cream preparation)

A gel cream preparation is prepared by the following formulation.

| (Components) | (Amount) |
|---|---|
| Mycophenolic acid | 0.5 |
| Crotamiton | 5.0 |
| Isopropyl myristate | 16.0 |
| Propylene glycol | 7.0 |
| Lauromacrogol | 1.5 |
| 4 % Aqueous carboxyvinyl polymer solution | 35.0 |
| 4 % L-arginine solution | 35.0 |
| | Totally 100.0 g |

Mycophenolic acid is dissolved in crotamiton, isopropyl myristate and lauromacrogol at about 70 to 80°C. Separately, to a 4 % aqueous carboxyvinyl polymer solution are added a 4 % aqueous L-arginine solution and propylene glycol, and the mixture is stirred to give a homogeneous gel, and thereafter, the mixture is warmed to about 70 to 80°C. To the mixture is added the above-prepared warmed solution containing the active ingredient. The mixture is emulsified and homogeneously mixed to give a gel cream preparation.

### Example 12 (Lotion)

A lotion is prepared by the following formulation.

| (Components) | (Amount) |
|---|---|
| Mycophenolic acid | 0.1 |
| Glycol salicylate | 1.0 |
| Self-emulsifiable monostearin | 2.0 |
| 1,3-Butylene glycol | 6.0 |
| Purified water | 90.9 |
| | Totally 100.0 g |

A part of purified water is gently warmed and thereto is added self-emulsifiable monostearin warmed at 60°C, and the mixture is stirred until it is completely dispersed. Separately, mycophenolic acid is dissolved in glycol salicylate and 1,3-butylene glycol with stirring. This mixture is added to the above warmed substrate with continuously stirring, and the mixture is stirred with cooling gradually. Purified water is added to the resulting mixture so as to make totally 100 g, and the mixture is mixed to give a lotion.

### Example 13 (Lotion)

A lotion is prepared by the following formulation.

| (Components) | (Amount) |
|---|---|
| Mycophenolic acid | 0.2 |
| Crotamiton | 2.0 |
| Methyl salicylate | 1.0 |
| Isopropyl myristate | 10.0 |
| Cetanol | 1.5 |
| Bleached beeswax | 0.3 |
| Purified lanolin | 1.0 |
| Polysorbate 80 | 6.0 |
| Lauromacrogol | 4.0 |
| Purified water | 74.0 |
| | Totally 100.0 g |

Mycophenolic acid is dissolved in crotamiton, methyl salicylate and isopropyl myristate by stirring at an elevated temperature, and to the solution are added cetanol, bleached beaswax, purified lanolin, polysorbate 80, and lauromacrogol, and the mixture is warmed and well mixed. Separately, purified water (about 50 g) is warmed. When both of the oily phase and aqueous phase become to 70 to 80°C, the aqueous phase is added to the oily phase while stirring continuously. After stirring the mixture, the remaining purified water is added thereto when the mixture becomes about 30°C so as to make totally 100 g to give a lotion.

### Example 14 (Solution preparation)

A solution preparation is prepared by the following formulation.

| (Components) | (Amount) |
|---|---|
| Mycophenolic acid | 2.0 |
| Crotamiton | 5.0 |
| Glycol salicylate | 5.0 |
| Polyethylene glycol 400 | 20.0 |
| 1,3-Butylene glycol | 40.0 |
| Purified water | q.s. |
| | Totally 100.0 ml |

Mycophenolic acid is dissolved in crotamiton, glycol salicylate, polyethylene glycol 400 and 1,3-butylene glycol with stirring well, and thereto is added purified water, and the mixture is well mixed and controlled so as to make totally 100 ml and filtered to give a solution preparation.

### Example 15 (Solution preparation)

A solution preparation is prepared by the following formulation.

| (Components) | (Amount) |
|---|---|
| Mycophenolic acid | 0.5 |
| Glycol salicylate | 5.0 |
| Ethanol | 40.0 |
| 1,3-Butylene glycol | 20.0 |
| Purified water | q.s. |
| | Totally 100.0 ml |

Mycophenolic acid is dissolved in glycol salicylate, ethanol and 1,3-butylene glycol with stirring well, and thereto is added purified water, and the mixture is well mixed and controlled so as to make totally 100 ml and filtered to give a solution preparation.

### Experiment 1

The stability of gel cream preparation was compared between the case of using crotamiton and the case of using sodium hydroxide.

### The preparation of the present invention A:

Mycophenolic acid (0.5 g) was homogeneously dissolved in crotamiton (5.0 g) with warming, and the mixture was kneaded together with the gel cream base as mentioned below to give a preparation (100 g).

### Reference preparation B:

Mycophenolic acid (0.5 g) was homogeneously dissolved in 5 % aqueous sodium hydroxide solution, and the mixture was kneaded together with the gel cream base as mentioned below to give a preparation (100 g).

### Gel cream base

| | |
|---|---|
| Isopropyl myristate | 16.0 |
| Lauromacrogol | 1.5 |
| Propylene glycol | 7.5 |
| 4 % Aqueous carboxyvinyl polymer solution | 37.5 |
| 4 % L-arginine solution | 37.5 |
| | Totally 100.0 g |

The above-mentioned Preparation A and Preparation B were kept at 90°C. After 8, 12 and 24 hours, the content of mycophenolic acid in the preparations was measured by liquid chromatography, and the ratio of the data to the initial content before keeping was calculated, and thereby the remaining ratio (%) was obtained. The results are shown in Table 1.

**Table 1**

| Tested preparations | Remaining ratio of mycophenolic acid | | |
|---|---|---|---|
| | 90°C - 8hr | 90°C - 12hr | 90°C - 24hr |
| Preparation A of the present invention | 100.2% | 100.5% | 100.7% |
| Reference Preparation B | 86.4% | 71.8% | 58.2% |

### Experiment 2

The stability of cream preparation was compared between the case of using crotamiton and the case of using sodium hydroxide.

### The preparation of the present invention C:

Mycophenolic acid (0.5 g) was homogeneously dissolved in crotamiton (5.0 g) with warming, and the mixture was kneaded together with the cream base as mentioned below to give a preparation (100 g).

### Reference preparation D:

Mycophenolic acid (0.5 g) was homogeneously dissolved in 5 % aqueous sodium hydroxide solution, and the mixture was kneaded together with the cream base as mentioned below to give a preparation (100 g).

### Cream base

| | |
|---|---|
| White vaseline | 45.0 |
| Cetanol | 20.0 |
| Sorbitan sesqui-oleate | 6.0 |
| Lauromacrogol | 1.0 |
| Purified water | 28.0 |
| | Totally 100.0 g |

The above-mentioned Preparation C and Preparation D were kept at 40°C. After 30 days and 60 days, the content of mycophenolic acid in the preparations was measured by liquid chromatography, and the remaining ratio (%) was calculated in the same manner as described in the above Experiment 1. The results are shown in Table 2.

**Table 2**

| Tested preparations | Remaining ratio of mycophenolic acid | |
|---|---|---|
| | 40°C - 30 days | 40°C - 60 days |
| Preparation C of the present invention | 99.5% | 99.7% |
| Reference Preparation D | 96.0% | 84.8% |

The preparations of Examples 1, 10 and 12 of the present invention were kept at 40°C or 60°C. After 30 days and 60 days, the remaining ratio (%) of mycophenolic acid was calculated in the same manner as described in the above Experiment 2. The results are shown in Table 3.

**Table 3**

| Tested preparations | Remaining ratio of mycophenolic acid | | | |
|---|---|---|---|---|
| | 40°C | | 60°C | |
| | 30days | 60days | 30days | 60days |
| Preparation of Example 1 | 99.7% | 100.4% | - | - |
| Preparation of Example 10 | 100.2% | 100.0% | 100.4% | 100.3% |
| Preparation of Example 12 | 99.5% | 100.2% | 100.3% | 101.2% |

The mycophenolic acid-containing composition for the epidermic application of the present invention is characteristic in that the active mycophenolic acid can be kept stably for a long period of time, and it can exhibit excellent effects for the treatment of various skin diseases, such as psoriasis, atopic dermatitis, alopecia areata by percutaneous application.

## Claims

1. A stable pharmaceutical composition for the epidermic application comprising as an active ingredient mycophenolic acid, which comprises incorporating one or more of a stabilizer selected from the group consisting of crotamiton, methyl salicylate and glycol salicylate.

2. The pharmaceutical composition for the epidermic application as claimed in claim 1, wherein mycophenolic acid is contained in an amount of 0.01 to 5.0 % by weight based on the whole weight of the composition.

3. The pharmaceutical composition for the epidermic application as claimed in claim 2, wherein mycophenolic acid is contained in an amount of 0.1 to 2.0 % by weight based on the whole weight of the composition.

4. The pharmaceutical composition for the epidermic application as claimed in any one of claims 1 to 3, wherein one or more of a stabilizer selected from the group consisting of crotamiton, methyl salicylate and glycol salicylate are contained in an amount of 1.0 to 15.0 % by weight based on the whole weight of the composition.

5. The pharmaceutical composition for epidermic application as claimed in any one of claims 1 to 4, which is in the form of a preparation selected from an ointment, a cream preparation, a gel preparation, a gel cream preparation, a lotion or a solution preparation.
